# EUROPEAN PATENT SPECIFICATION

(11) **EP 4 412 612 B1**
(45) Date of publication and mention of the grant of the patent: **06.08.2025**
(21) Application number: 22826188.9
(22) Date of filing: 06.10.2022
(51) Int. Cl.: A61K 31/496, A61P 35/00, A61P 35/02, A61K 45/06

(54) **METHODS OF TREATING CANCER USING HSF1 PATHWAY INHIBITORS**
VERFAHREN ZUR BEHANDLUNG VON KREBS MIT HSF1-PFAD-HEMMERN
MÉTHODES DE TRAITEMENT DU CANCER À L'AIDE D'INHIBITEURS DE LA VOIE HSF1

(30) Priority: 06.10.2021 US 202163252657 P
(43) Date of publication of application: 14.08.2024
(73) Proprietor: Institute of Cancer Research, London SW7 3RP (GB)
(72) Inventor: WORKMAN, Paul, Surrey RH5 6JF (GB); CLARK, Paul Andrew, London SW6 4EP (GB); TE POELE, Robert Herman, Purley CR8 3EE (GB)
(74) Representative: Kilburn & Strode LLP
(86) International application number: PCT/IB2022/000591
(87) International publication number: WO 2023/057819

(56) References cited:
- WO-A1-2015/049535
- MATTHEW D. CHEESEMAN ET AL: "Discovery of a Chemical Probe Bisamide (CCT251236): An Orally Bioavailable Efficacious Pirin Ligand from a Heat Shock Transcription Factor 1 (HSF1) Phenotypic Screen", JOURNAL OF MEDICINAL CHEMISTRY, vol. 60, no. 1, 12 January 2017 (2017-01-12), US, pages 180 - 201, XP055391134, ISSN: 0022-2623, DOI: 10.1021/acs.jmedchem.6b01055
- BEAUFORT CORINE M. ET AL: "Ovarian Cancer Cell Line Panel (OCCP): Clinical Importance of In Vitro Morphological Subtypes", PLOS ONE, vol. 9, no. 9, 17 September 2014 (2014-09-17), pages 1 - 16, XP093020479, DOI: 10.1371/journal.pone.0103988
- ANONYMOUS: "United States Security and Exchange Commission - Nuvectis Pharma Inc", 14 January 2022 (2022-01-14), pages 1 - 178, XP093020518, Retrieved from the Internet <URL:https://www.sec.gov/Archives/edgar/data/1875558/000110465922004520/tm2134024-7_s1a.htm>

## Description

### CROSS-REFERENCE TO RELATED APPLICATIONS

This application claims the benefit of, and priority to, U.S. S.N. 63/252,657 filed

### BACKGROUND

Cancer is caused by uncontrolled and unregulated cellular proliferation. Precisely what causes a cell to become malignant and proliferate in an uncontrolled and unregulated manner has been the focus of intense research over recent decades. This research has led to the identification of a number of molecular targets associated with key metabolic pathways that are known to be associated with malignancy.

Heat shock factor 1 pathway (HSF1 pathway) is one target pathway that is of interest. HSF1 is the master regulator of the heat shock response, in which multiple genes are induced in response to temperature increase and other stresses. At non-shock temperatures in humans and other vertebrates, HSF1 is produced constitutively, but is inactive and bound by protein HSP90. At an elevated temperature, HSF1 is released by HSP90, moves from the cytoplasm to the nucleus, and trimerizes. This active HSF1 form binds to sequences called heat shock elements (HSE) in DNA and activates transcription of heat shock genes by RNA polymerase II. During cessation of the heat shock response, HSF 1 is phosphorylated by mitogen -activated protein kinases (MAPKs) and glycogen synthase kinase 3 (GSK3) and returns to an inactive state.

HSF1 pathway activity has been implicated in several diseases, including cancer, and autoimmune, and viral diseases. HSF1 and other heat shock proteins (whose expression is increased by HSF1) are over-expressed in, or have otherwise been implicated in, breast, endometrial, fibrosarcoma, gastric, kidney, liver, lung, lymphoma, neuroectodermal, neuroblastoma, Ewing's sarcoma, prostate, skin, squamous cell, and testicular cancers, leukemia (e.g., promyelocytic leukemia), and Hodgkin's disease.

ARID1A is a member of the SWI/SNF family, whose members have helicase and ATPase activities and are thought to regulate transcription of certain genes by altering the chromatin structure around those genes. ARID1A controls the expression of key components of the metabolic pathway leading to increased cancer cell survival by enhanced generation of glutathione (GSH) and protection from metabolic stress induced by reactive oxygen species (ROS).

ARID1A is mutated in multiple solid tumor types at varying frequencies. For example, ARID1A mutations are commonly found to be present across cancer and solid tumor types including, for example, breast, lung, esophageal, pancreatic, urothelial, uterine, ovarian, gastrointestinal and liver. ARID1A mutations are associated with an aberrant cell cycle leading to reduced GSH levels, which results in decreased protection against stress. The cancer cells compensate for this by overactivation of the HSF-1 pathway.

Protein loss associated with ARID1A mutations can be used as a patient selection strategy in solid tumor types. Methodologies to detect the ARID1A mutations are known in the art. Accordingly, there is a need for methods for the treatment of diseases or conditions in which HSF1 pathway activity is mediated, e.g., for the treatment cancers manisfested as solid tumors in which ARID1A1a mutations are present.

Matthew D. Cheeseman et. al describe the discovery of a new chemical probe, bisamide (CCT251236), identified using an unbiased phenotypic screen to detect inhibitors of the HSF1 stress-pathway (Cheeseman, Matthew D., et al. "Discovery of a chemical probe bisamide (CCT251236): an orally bioavailable efficacious pirin ligand from a heat shock transcription factor 1 (HSF1) phenotypic screen." Journal of medicinal chemistry 60.1 (2017): 180-201).

### SUMMARY

The references to methods of treatment by therapy of this description are to be interpreted as references to compounds, pharmaceutical compositions and medicaments of the present invention for use in those methods.

The present invention is defined by the appended claims.

The present disclosure is directed, at least in part, to methods of treating cancer using compounds that modulate, e.g., inhibit, the HSF1 pathway. For example, disclosed herein is a method of treating a cancer in a patient in need thereof, comprising administering to the patient an effective amount of a HSF1 pathway inhibitor, wherein the cancer comprises solid tumors harboring an ARID1A mutation, for example, solid tumors identified as having an ARID1A mutation. In some embodiments, the cancer may be selected from the group consisting of, for example, an ovarian cancer, stomach cancer and pancreatic cancer.

Further disclosed herein is a method of treating an ovarian cancer in a patient in need thereof, comprising administering to the patient an effective amount of a HSF1 pathway inhibitor, wherein the cancer comprises solid tumors harboring an ARID1A mutation, for example, solid tumors identified as having an ARID1A mutation. In some embodiments, the ovarian cancer is, for example, endometrioid ovarian cancer or ovarian clear cell carcinoma.

According to the present invention, the HSF1 pathway inhibitor is *N*-(5-(2,3-dihydrobenzo[b][1,4]dioxine-6-carboxamido)-2-fluorophenyl)-2-((4-ethylpiperazin-1-yl)methyl)quinoline-6-carboxamide, or a pharmaceutically acceptable salt thereof, and is represented by:

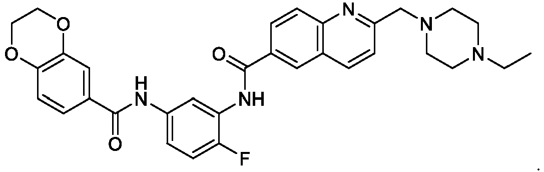

For example, disclosed herein is a method of treating an ovarian cancer in a patient in need thereof, comprising administering to the patient an effective amount of N-(5-(2,3-dihydrobenzo[b][1,4]dioxine-6-carboxamido)-2-fluorophenyl)-2-((4-ethylpiperazin-1-yl)methyl)quinoline-6-carboxamide, or a pharmaceutically acceptable salt thereof, wherein the cancer comprises solid tumors harboring an ARID1A mutation.

### BRIEF DESCRIPTION OF THE DRAWINGS

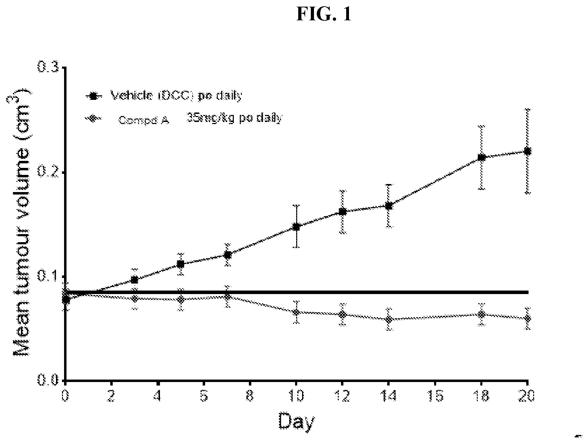

FIG. 1 depicts the antitumor activity (e.g., tumor growth inhibition) of Compound A in an ovarian cancer xenograft model (SKOV-3). The tumor was identified as harboring an ARID1A mutation. Cr nude mice were injected with 5 × 10⁶ SK-OV-3 cells and, once the tumour was established, administered vehicle control (DCC) or 35 mg/kg Compound A in DCC once daily (or as described in the text) by oral gavage. Graph shows mean tumour volume over the dosing period of the study; error bars represent standard error of the mean. Control and 35 mg/kg group n=10. Horizontal black line is 35 mg/kg group tumour volume on Day 0.

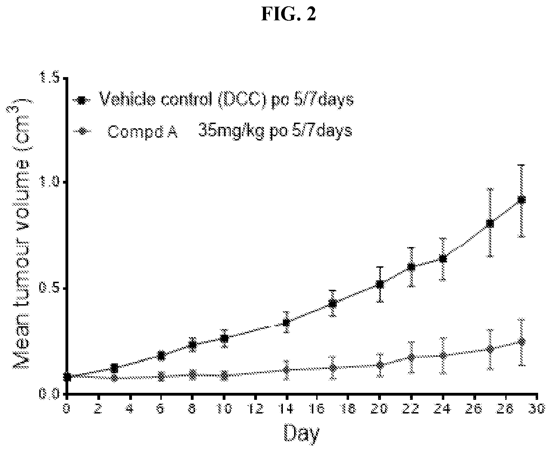

FIG. 2 depicts the antitumor activity (e.g., tumor growth inhibition) of Compound A in an ovarian cancer xenograft model (TOV-21G). The tumor was identified as harboring an ARID1A mutation. NCr nude mice were injected with 3 × 10⁶ TOV-21G cells and, once the tumour was established, administered vehicle control (DCC) or 35 mg/kg Compound A in DCC once daily for 5 days out of every 7 by oral gavage. Graph shows mean tumour volume over the dosing period of the study; error bars represent standard error of the mean. Vehicle control group n=8 and 35 mg/kg dose group n=11.

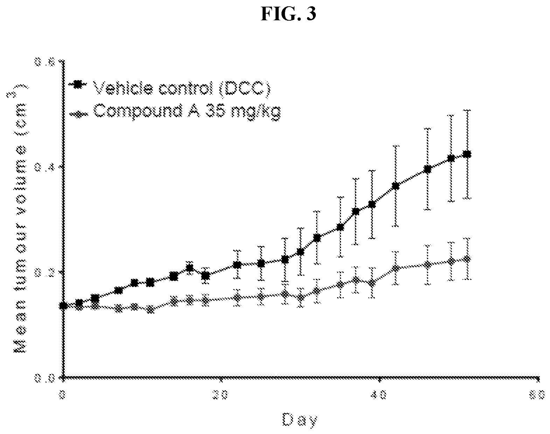

FIG. 3 depicts the antitumor activity (e.g., tumor growth inhibition) of Compound A in an ovarian cancer xenograft model (OVISE). NCr nude mice were injected with 5 × 10⁶ OVISE cells and, once the tumour was established, administered vehicle control (DCC) or 35 mg/kg Compound A in DCC once daily for 5 days out of every 7 by oral gavage. Graph shows mean tumour volume over the dosing period of the study; error bars represent standard error of the mean. Vehicle control group n=10 and 35 mg/kg Compound A group n=11.

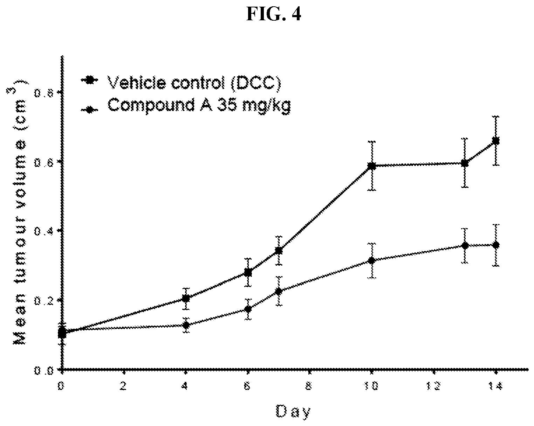

FIG. 4 depicts the antitumor activity (e.g., tumor growth inhibition) of Compound A in an ovarian cancer xenograft model (IGROV-1). NCr nude mice were injected with 3 × 10⁶ IGROV-1 cells and, once the tumour was established, administered vehicle control (DCC) or 35 mg/kg Compound A in DCC once daily by oral gavage in an intermittent schedule. Graph shows mean tumour volume over the dosing period of the study; error bars represent standard error of the mean. Vehicle control group n=5 and 35 mg/kg Compound A group n=6.

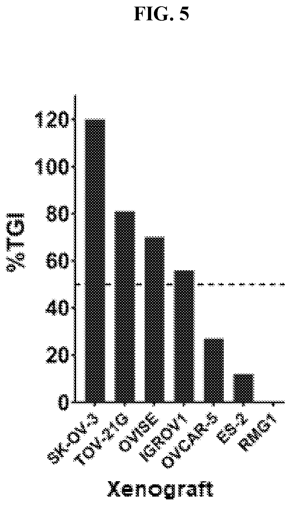

FIG. 5 depicts a summary of tumour xenograft experiments. Established tumour xenografts were treated (35 mg/kg Compound A oral schedule as in Table 1 once daily as indicated). TGI>50% was considered significant and is indicated by the dotted line. All experiments were conducted with at least n=9 mice control and treated (except IGROV-1 n=6 treated and n=5 control).

### DETAILED DESCRIPTION

The features and other details of the disclosure will now be more particularly described. Before further description of the present disclosure, certain terms employed in the specification, examples and appended claims are collected here. These definitions should be read in light of the remainder of the disclosure and as understood by a person of skill in the art. Unless defined otherwise, all technical and scientific terms used herein have the same meaning as commonly understood by a person of ordinary skill in the art.

### Definitions

"Treating" includes any effect, e.g., lessening, reducing, modulating, or eliminating, that results in the improvement of the condition, disease, disorder and the like. FOr example, "treating" or "treatment" of a state, disorder or condition therefore includes: ( 1 ) preventing or delaying the appearance of clinical symptoms of the state, disorder or condition developing in a human that may be afflicted with or predisposed to the state, disorder or condition but does not yet experience or display clinical or subclinical symptoms of the state, disorder or condition, (2) inhibiting the state, disorder or condition, i.e., arresting, reducing or delaying the development of the disease or a relapse thereof (in case of maintenance treatment) or at least one clinical or subclinical symptom thereof, or (3) relieving or attenuating the disease, i.e., causing regression of the state, disorder or condition or at least one of its clinical or subclinical symptoms. It is to be appreciated that references to "treating" or "treatment" include prophylaxis as well as the alleviation of established symptoms of a condition.

The term "disorder" refers to and is used interchangeably with, the terms "disease," "condition," or "illness," unless otherwise indicated.

"Pharmaceutically or pharmacologically acceptable" include molecular entities and compositions that do not produce an adverse, allergic or other untoward reaction when administered to an animal, or a human, as appropriate. For human administration, preparations should meet sterility, pyrogenicity, and general safety and purity standards as required by FDA Office of Biologics standards.

"Individual," "patient," or "subject" are used interchangeably and include any animal, including mammals, preferably mice, rats, other rodents, rabbits, dogs, cats, swine, cattle, sheep, horses, or primates, and most preferably humans. The compounds of the present disclosure can be administered to a mammal, such as a human, but can also be administered to other mammals such as an animal in need of veterinary treatment, e.g., domestic animals (e.g., dogs, cats, and the like), farm animals (e.g., cows, sheep, pigs, horses, and the like) and laboratory animals (e.g., rats, mice, guinea pigs, and the like). The mammal treated in the methods of the present disclosure is desirably a mammal in which treatment, for example, of a cancer or a blood disorder is desired. "Modulation" includes antagonism (e.g., inhibition), agonism, partial antagonism and/or partial agonism.

In the present specification, the terms "effective amount" or "therapeutically effective amount" means the amount of the subject compound that will elicit the biological or medical response of a tissue, system or animal, (e.g. mammal or human) that is being sought by the researcher, veterinarian, medical doctor or other clinician. The compounds of the present disclosure are administered in therapeutically effective amounts to treat a disease. Alternatively, a therapeutically effective amount of a compound is the quantity required to achieve a desired therapeutic and/or prophylactic effect.

The term "pharmaceutically acceptable salt(s)" as used herein refers to salts of basic groups that may be present in compounds used in the compositions. Compounds included in the present compositions that are basic in nature are capable of forming a wide variety of salts with various inorganic and organic acids.

The term "and/or" is used in this disclosure to mean either "and" or "or" unless indicated otherwise.

As used herein, the words "a" and "an" are meant to include one or more unless otherwise specified. For example, the term "an agent" encompasses both a single agent and a combination of two or more agents.

Where the use of the term "about" is before a quantitative value, the present disclosure also includes the specific quantitative value itself, unless specifically stated otherwise. As used herein, the term "about" refers to a ± 10% variation from the nominal value unless otherwise indicated or inferred.

### Methods

In some embodiments, disclosed herein is a method of treating a cancer in a patient in need thereof, comprising administering to the patient an effective amount of a HSF1 pathway inhibitor, wherein the cancer comprises solid tumors harboring an ARID1A mutation.

For example, in some embodiments, the cancer is selected from the group consisting of, for example, an ovarian cancer, stomach cancer and pancreatic cancer. In certain embodments, the cancer is endometrioid ovarian cancer, for example, the cancer is relapsed or refractory endometrioid ovarian cancer. In other embodiments, the cancer is ovarian clear cell carcinoma. In some emodiments, the cancer is relapsed or refractory ovarian clear cell cancer. In certain embodiments, the ovarian cancer is substantially resistant to platinum-based chemotherapy, taxane-based chemotherapy, or a combination thereof.

In the context of the present invention, the HSF1 pathway inhibitor is 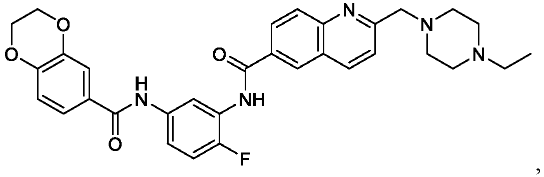 or a pharmaceutically acceptable salt thereof.

For example, in certain embodiments the HSF1 pathway inhibitor is

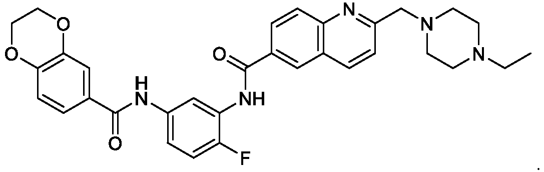

In some embodiments, the ARID1A mutation is a heterozygous ARID1A mutation. In other embodiments, the ARID1A mutation is a homozygous ARID1A mutation.

In further embodiments, the HSF1 pathway inhibitor is administered orally or subcutaneously. In some embodiments, the administration results in, for example, about a 2-5 fold decrease in the mean GI₅₀ in ARID1A mutant cancer cells compared to wild type ARID1A cells. In other embodiment, the administration results in, for example, about a 4-5 fold decrease in the mean GI₅₀ in ARID1A mutant ovarian cancer cells compared to wild type ARID1A ovarian cells.

In some embodiments, the method further and optionally comprises administering one or more additional cancer chemotherapeutic agents. For example, in other embodiments, the method further and optionally comprises administering an additional cancer chemotherapeutic agent.

Also disclosed herein is a method of treating an ovarian cancer in a patient in need thereof, comprising administering to the patient an effective amount of a HSF 1 pathway inhibitor, wherein the cancer comprises solid tumors harboring an ARID1A mutation. In certain embodments, the cancer is endometrioid ovarian cancer, for example, the cancer is relapsed or refractory endometrioid ovarian cancer. In other embodiments, the cancer is ovarian clear cell carcinoma. In some emodiments, the cancer is relapsed or refractory ovarian clear cell cancer. In certain embodiments, the HSF1 pathway inhibitor is: 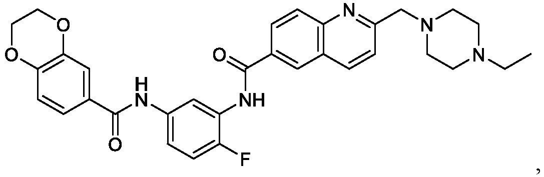 or a pharmaceutically acceptable salt thereof.

Further disclosed herein is a method of treating an ovarian cancer in a patient in need thereof, comprising administering to the patient an effective amount of a HSF1 pathway inhibitor represented by 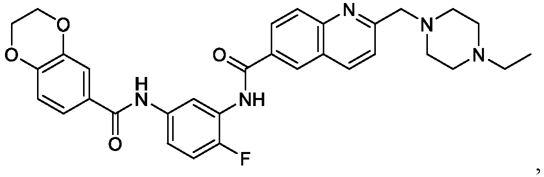
or a pharmaceutically acceptable salt thereof,
wherein the cancer comprises solid tumors harboring an ARID1A mutation. In some embodiments, the ovarian cancer is, for example, endometrioid ovarian cancer or ovarian clear cell carcinoma.

In some embodiments, a cancer type or solid tumor type disclosed herein has been identified as having an an ARID1A mutation. Methodologies to detect the ARID1A mutations are known in the art. For example, protein loss associated with ARID1A mutations can be used as a patient selection strategy in solid tumor types.

In particular, in certain embodiments, the disclosure provides a method of treating the above medical indications comprising administering to a patient in need thereof an effective amount of a HSF1 pathway inhibitor disclosed herein. In certain other embodiments, the disclosure provides a method of treating the above medical conditions in a patient in need thereof, comprising orally, subcutaneously, or intravenously administering to the patient a composition comprising a disclosed HSF1 pathway inhibitor.

In some embodiments, a method of treatment disclosed herein may produce an antiproliferative effect. In some embodiments, a method of treatment disclosed herein may be a method of treating a proliferative disease or a proliferative disorder. The terms "proliferative disease" and "proliferative disorder" are used interchangeably herein and pertain to an unwanted or uncontrolled cellular proliferation of excessive or abnormal cells which is undesired, such as, neoplastic or hyperplasticgrowth, whether in vitro or in vivo. Examples of proliferative conditions include, but are not limited to, pre-malignant and malignant cellular proliferation, including but not limited to, cancers and solid tumors. Any type of cell may be treated, including but not limited to, lung, liver, stomach, colon, breast, ovarian, prostate, liver, pancreas, brain, and skin.

The anti-proliferative effects of the methods disclosed herein have particular application in the treatment of human cancers by virtue of their HSF 1 inhibitory properties. The anti-cancer effect may arise through one or more mechanisms, including but not limited to, the regulation of cell proliferation, the inhibition of angiogenesis (the formation of new blood vessels), the inhibition of metastasis (the spread of a tumour from its origin), the inhibition of invasion (the spread of tumour cells into neighbouring normal structures), or the promotion of apoptosis (programmed cell death). The anti-proliferative and anti-cancer effects may be observed in cancer and solid tumor types identified as having an ARID1A mutation.

The treatments defined herein may be applied as a sole therapy or may involve, in addition to the compound of the invention, conventional surgery, radiotherapy, gene therapy or therapy with a chemotherapeutic agent or a molecularly targeted agent. Such conjoint treatment may be achieved by way of the simultaneous, sequential or separate dosing of the individual components of the treatment. The term "combination" as used herein refers to simultaneous, separate or sequential administration. In some embodimens, "combination" refers to simultaneous administration. In other embodiments, "combination" refers to separate administration. In further embodiments, "combination" refers to sequential administration. Where the administration is sequential or separate, the delay in administering the second component should not be such as to lose the beneficial effect of the combination.

### EXAMPLES

The following non-limiting examples illustrate the disclosure.

*N*-(5-(2,3-Dihydrobenzo[b][1,4]dioxine-6-carboxamido)-2-fluorophenyl)-2-((4-ethylpiperazin-1-yl)methyl)quinoline-6-carboxamide (Compound A) can be prepared according to the synthetic procedures in WO 2015/049535.

### In Vivo Efficacy Studies:

The *in vivo* efficacy of Compound A was investigated in xenograft tumours in athymic nude mice with a focus on ovarian cancers. The efficacy of Compound A was investigated in xenograft bearing mice with the following ovarian cancer cell lines: SK-OV-3; TOV-21G, OVISE, IGROV1, OVCAR-5, ES-2, and RMG1. Of the seven cell lines, SK-OV-3; TOV-21G, OVISE, and IGROV1 were identified as harboring an ARID1A mutation, while OVCAR-5, ES-2, and RMG1 were ARID1A wildtype.

Once the tumours were established, mice were randomised into vehicle control and treatment groups. Mice in both the vehicle control and treatment groups that were removed during the course of the study due to spontaneous or treatment derived tumour regression, tumour growth approaching licence limits, tumour ulceration or loss of condition were excluded from the final analysis of the mean tumour volume and standard error of the mean and up to the point they were removed from the study. The number of mice undergoing complete tumour regression in the treatment group is noted where applicable.

Two groups of 12 female NCr nude mice (3 groups of 10 for SK-OV-3) were injected with 3-5 × 10⁶ cells of one of the above cancer cell lines, subcutaneously. Once the tumours had reached this size the groups were administered vehicle control (10% DMSO, 90% of 25% 2-hydroxypropyl-beta-cyclodextrin in 50 mM sodium citrate buffer pH5 [DCC]) or Compound A at 35 or 70 mg/kg orally in DCC once daily by gavage. Body weights were taken daily and animals were monitored for signs of toxicity. To evaluate drug levels the animals were euthanised in batches of 3 to 4 at 2, 6 and 24 hours after the last dose and terminal plasma and tumour samples collected for PK analysis. The difference in final tumour volume (p<0.005, Mann-Whitney test) in the Compound A dose group relative to the control group was recorded. The results are shown in Table 1.

**Table 1. Details of the 35 mg/kg Compound A dose schedule and TGIs obtained in the studies**

| Xenograft | Compound A regime | Doses given / no. of study days | TGI (%) |
|---|---|---|---|
| SK-OV-3 | Continuous | 20/20 | 120 |
| TOV-21G | 5/7 days | 21/19 | 81 |
| OVISE | 5/7 days | 38/52 | 70 |
| IGROV1 | Intermittent | 11/15 | 56 |
| OVCAR-5 | 5/7 days | 21/27 | 27 |
| ES-2 | 5/7 days | 12/17 | 12 |
| RMG1 | 5/7 days | 15/19 | -3.0 |

FIG. 1, FIG. 2, FIG. 3, and FIG. 4 depicts the antitumor activity (e.g., tumor growth inhibition) of Compound A in ovarian clear cell cancer xenograft models: SKOV-3, TOV-21G, OVISE, and IGROV1, each identified as harboring an ARID1A mutation.

As shown in Table 1, a small signal of efficacy was seen in the ARID1A wild type OVCAR-5 and ES-2 models (%TGI of 27% and 12%, respectively), however this did not meet the criteria for efficacy (%TGI.50%). No inhibition of xenograft tumour growth was seen in the ARID1A wildtype RMG1 model. Plasma Cₘₐₓ in the OVCAR-5 and ES-2 models were within the range seen in successful efficacy studies. while the levels in the RMG1 model was less than half the lowest level seen in successful efficacy studies. The ARID1A mutated, SK-OV-3 ovarian cancer cell line was the most sensitive cell line in xenograft models.

FIG. 5 depicts a comparative graph of the antitumor activity (e.g., tumor growth inhibition) of Compound A in the seven ovarian cancer xenograft models studied: SK-OV-3; TOV-21G, OVISE, IGROV1, OVCAR-5, ES-2, and RMG1.

### EQUIVALENTS

While specific embodiments of the subject disclosure have been discussed, the above specification is illustrative and not restrictive. Many variations of the disclosure will become apparent to those skilled in the art upon review of this specification. The invention is defined by the claims.

Unless otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in this specification and attached claims are approximations that may vary depending upon the desired properties sought to be obtained by the present disclosure.

## Claims

1. A compound for use in a method of treating a cancer in a patient in need thereof, the method comprising administering to the patient an effective amount of the compound;
wherein the compound is represented by: 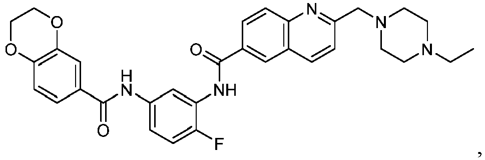 or a pharmaceutically acceptable salt thereof; and
wherein the cancer comprises solid tumors harboring an ARID1A mutation.

2. The compound for use of claim 1, wherein the cancer is selected from the group consisting of an ovarian cancer, stomach cancer and pancreatic cancer.

3. The compound for use of claim 1 or 2, wherein the cancer is endometrioid ovarian cancer.

4. The compound for use of any one of claims 1-3, wherein the cancer is relapsed or refractory endometrioid ovarian cancer.

5. The compound for use of claim 1 or 2, wherein the cancer is ovarian clear cell carcinoma.

6. The compound for use of claim 1, 2 or 5, wherein the the cancer is relapsed or refractory ovarian clear cell cancer.

7. The compound for use of any one of claims 1-6, wherein the ovarian cancer is substantially resistant to platinum-based chemotherapy, taxane-based chemotherapy, or a combination thereof.

8. The compound for use of any one of claims 1-7, wherein the compound is

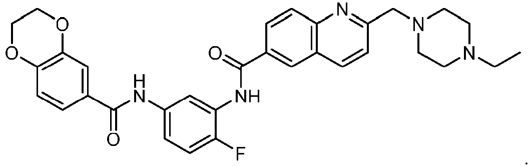

9. The compound for use of any one of claims 1-8, wherein the ARID1A mutation is a heterozygous ARID1A mutation.

10. The compound for use of any one of claims 1-8, wherein the ARID1A mutation is a homozygous ARID1A mutation.

11. The compound for use of any one of claims 1-10, wherein the compound is administered orally or subcutaneously.

12. The compound for use of any one of claims 1-11, wherein the method further and optionally comprises administering one or more additional cancer chemotherapeutic agents.

13. The compound for use of any one of claims 1-12, wherein the method further and optionally comprises administering an additional cancer chemotherapeutic agent.

## Patentansprüche

1. Verbindung zur Verwendung bei einem Verfahren zur Behandlung einer Krebserkrankung bei einem Patienten, bei dem diesbezüglicher Bedarf besteht, wobei das Verfahren das Verabreichen einer wirksamen Menge der Verbindung an den Patienten umfasst;
wobei die Verbindung wiedergegeben wird durch: 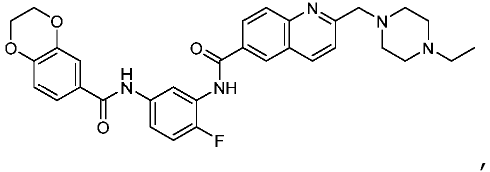 oder ein pharmazeutisch unbedenkliches Salz davon; und
wobei die Krebserkrankung solide Tumoren, die eine ARID1A-Mutation beherbergen, umfasst.

2. Verbindung zur Verwendung nach Anspruch 1, wobei die Krebserkrankung aus der Gruppe bestehend aus Eierstockkrebs, Magenkrebs und Bauchspeicheldrüsenkrebs ausgewählt ist.

3. Verbindung zur Verwendung nach Anspruch 1 oder 2, wobei es sich bei der Krebserkrankung um endometrioiden Eierstockkrebs handelt.

4. Verbindung zur Verwendung nach einem der Ansprüche 1-3, wobei es sich bei der Krebserkrankung um rezidivierten oder refraktären endometrioiden Eierstockkrebs handelt.

5. Verbindung zur Verwendung nach Anspruch 1 oder 2, wobei es sich bei dem Krebs um klarzelliges Ovarialkarzinom handelt.

6. Verbindung zur Verwendung nach Anspruch 1, 2 oder 5, wobei es sich bei der Krebserkrankung um rezidivierten oder refraktären klarzelligen Eierstockkrebs handelt.

7. Verbindung zur Verwendung nach einem der Ansprüche 1-6, wobei der Eierstockkrebs weitgehend resistent gegenüber platinbasierter Chemotherapie, taxanbasierter Chemotherapie oder einer Kombination davon ist.

8. Verbindung zur Verwendung nach einem der Ansprüche 1-7, wobei es sich bei der Verbindung um 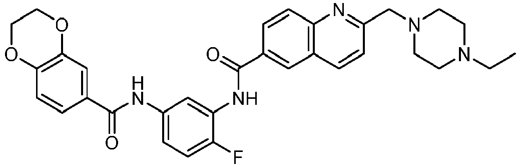 handelt.

9. Verbindung zur Verwendung nach einem der Ansprüche 1-8, wobei es sich bei der ARID1A-Mutation um eine heterozygote ARID1A-Mutation handelt.

10. Verbindung zur Verwendung nach einem der Ansprüche 1-8, wobei es sich bei der ARID1A-Mutation um eine homozygote ARID1A-Mutation handelt.

11. Verbindung zur Verwendung nach einem der Ansprüche 1-10, wobei die Verbindung oral oder subkutan verabreicht wird.

12. Verbindung zur Verwendung nach einem der Ansprüche 1-11, wobei das Verfahren ferner und gegebenenfalls das Verabreichen eines oder mehrerer zusätzlicher Krebs-Chemotherapeutika umfasst.

13. Verbindung zur Verwendung nach einem der Ansprüche 1-12, wobei das Verfahren ferner und gegebenenfalls das Verabreichen eines zusätzlichen Krebs-Chemotherapeutikums umfasst.

## Revendications

1. Composé pour utilisation dans un procédé de traitement d'un cancer chez un patient qui en a besoin, le procédé comprenant l'administration au patient d'une quantité efficace du composé ;
dans lequel le composé est représenté par : 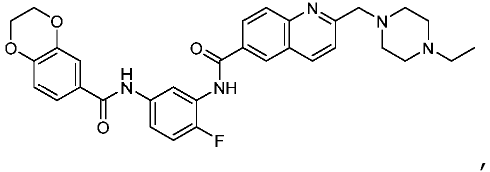 ou un sel pharmaceutiquement acceptable de celui-ci ; et
dans lequel le cancer comprend des tumeurs solides hébergeant une mutation ARID1A.

2. Composé pour utilisation selon la revendication 1, dans lequel le cancer est choisi dans le groupe constitué par un cancer de l'ovaire, un cancer de l'estomac et un cancer du pancréas.

3. Composé pour utilisation selon la revendication 1 ou 2, dans lequel le cancer est un cancer ovarien endométrioïde.

4. Composé pour utilisation selon l'une quelconque des revendications 1 à 3, dans lequel le cancer est un cancer ovarien endométrioïde récidivant ou réfractaire.

5. Composé pour utilisation selon la revendication 1 ou 2, dans lequel le cancer est un carcinome ovarien à cellules claires.

6. Composé pour utilisation selon la revendication 1, 2 ou 5, dans lequel le cancer est un cancer ovarien à cellules claires récidivant ou réfractaire.

7. Composé pour utilisation selon l'une quelconque des revendications 1 à 6, dans lequel le cancer de l'ovaire est sensiblement résistant à une chimiothérapie à base de platine, à une chimiothérapie à base de taxane, ou à une combinaison de celles-ci.

8. Composé pour utilisation selon l'une quelconque des revendications 1 à 7, dans lequel le composé est

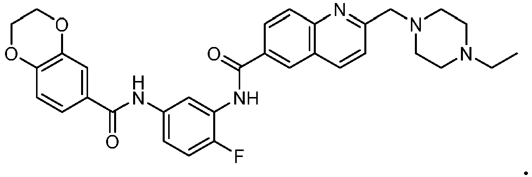

9. Composé pour utilisation selon l'une quelconque des revendications 1 à 8, dans lequel la mutation ARID1A est une mutation ARID1A hétérozygote.

10. Composé pour utilisation selon l'une quelconque des revendications 1 à 8, dans lequel la mutation ARID1A est une mutation ARID1A homozygote.

11. Composé pour utilisation selon l'une quelconque des revendications 1 à 10, dans lequel le composé est administré par voie orale ou sous-cutanée.

12. Composé pour utilisation selon l'une quelconque des revendications 1 à 11, dans lequel le procédé comprend en outre et éventuellement l'administration d'un ou plusieurs agents chimiothérapeutiques anticancéreux supplémentaires.

13. Composé pour utilisation selon l'une quelconque des revendications 1 à 12, dans lequel le procédé comprend en outre et éventuellement l'administration d'un agent chimiothérapeutique anticancéreux supplémentaire.
